# EUROPÄISCHE PATENTANMELDUNG

(11) **EP 2 915 583 A1**
(43) Veröffentlichungstag der Anmeldung: **09.09.2015**
(21) Anmeldenummer: 15156899.5
(22) Anmeldetag: 27.02.2015
(51) Int. Cl.: B01J 23/40, B01J 23/48, B01J 23/72, B01J 35/00, B82Y 30/00

(54) **Mit katalytisch wirksamen Partikeln belegter Katalysatorträger, Verfahren zu dessen Herstellung und Verfahren zur Katalyse unter Nutzung von Plasmonenresonanz**

(30) Priorität: 28.02.2014 DE 102014102741
(71) Anmelder: JENOPTIK Katasorb GmbH, 07745 Jena (DE)
(72) Erfinder: Krech, Thomas, 07743 Jena (DE); Krippendorf, Ronald, 07743 Jena (DE); Garwe, Frank, 99817 Eisenach (DE); Müller, Robert, 07749 Jena (DE)
(74) Vertreter: Schaller, Renate

(57) **Zusammenfassung**

Die Erfindung betrifft einen mit katalytisch wirksamen Partikeln (3) belegten Katalysatorträger (1), ein Verfahren zu dessen Herstellung sowie ein Verfahren zur Katalyse unter Nutzung von Plasmonenresonanz. Die Plasmonenresonanz wird durch Licht (5) einer Anregungswellenlänge angeregt, das auf die Partikel (3) gestrahlt ist. Ein erfindungsgemäßer Katalysatorträger (1) ist ein mit katalytisch wirksamen Partikeln (3) belegter Katalysatorträger (1), wobei das Material und die Form der Partikel (3) dazu geeignet ist, bei Einwirkung von Licht (5) einer bestimmten Anregungswellenlänge und Intensität auf der Oberfläche der Partikel (3) Plasmonenresonanz und hohe Feldüberhöhungen hervorzurufen, dadurch gekennzeichnet, dass der Katalysatorträger (1) aus einem Trägermaterial besteht, das für die Anregungswellenlänge transparent ist und durch das Trägermaterial eine Porosität bereitgestellt ist und die Porosität offenporig ist, wobei freie Oberflächen der Poren (2) mindestens bereichsweise mit Partikeln (3) belegt sind.

## Beschreibung

Die Erfindung betrifft einen mit katalytisch wirksamen Partikeln belegten Katalysatorträger, ein Verfahren zu dessen Herstellung sowie ein Verfahren zur Durchführung mindestens einer katalytischen Reaktion unter Verwendung eines mit katalytisch wirksamen Partikeln belegten Katalysatorträgers.

Aus der Publikation von Linic et al. (Linic, S.; Christopher, P; Xin, H.; Marimuthu, A. 2013, Catalytic and photocatalytic nanoparticles with targeted geometric and plasmonic properties, Accounts of chemical research, 46 (8): 1890-1899) ist die Möglichkeit der Verwendung von Edelmetall-Nanopartikeln verschiedener Größe und Form zur heterogenen Katalyse beschrieben. Dazu werden Silberpartikel mit Licht einer Anregungswellenlänge bestrahlt und an deren Oberfläche Plasmonenresonanzen angeregt. Die so angeregten Partikel dienen zur Durchführung katalytischer Reaktionen bei vergleichsweise niedrigen Temperaturen.

Der Erfindung liegt die Aufgabe zugrunde, eine weitere Möglichkeit zur Durchführung einer Katalyse unter Nutzung von Plasmonenresonanz vorzuschlagen. Der Erfindung liegt ferner die Aufgabe zugrunde, technische Maßnahmen sowie einen Katalysatorträger vorzuschlagen, durch die bzw. durch den eine Katalyse auf effektive Weise durchführbar ist.

Die Aufgaben werden in den unabhängigen Ansprüchen gelöst. Die abhängigen Ansprüche geben vorteilhafte Ausgestaltungen an.

Die Aufgabe wird durch einen mit katalytisch wirksamen Partikeln belegten Katalysatorträger gelöst, wobei das Material und die Form der Partikel dazu geeignet ist, bei Einwirkung von Licht einer bestimmten Anregungswellenlänge und Intensität auf der Oberfläche der Partikel Plasmonenresonanz und hohe Feldüberhöhungen hervorzurufen, wobei der Katalysatorträger aus einem Trägermaterial besteht, das für die Anregungswellenlänge transparent ist, durch das Trägermaterial eine Porosität bereitgestellt ist und die Porosität offenporig ist, wobei freie Oberflächen der Poren mindestens bereichsweise mit Partikeln belegt sind.

Unter Partikeln im Sinne dieser Beschreibung werden sowohl einzelne Körper, beispielsweise einzelne Körner des Materials, als auch Agglomerate solcher Körper (partikuläre Agglomerate) verstanden. In einer bevorzugten Ausführung des erfindungsgemäßen belegten Katalysatorträgers sind die Partikel sogenannte Nanopartikel. Im Sinne dieser Beschreibung sind Partikel vorzugsweise Nanopartikel.

Nanopartikel sind Partikel und partikuläre Agglomerate, deren mittlerer Durchmesser oder deren Ausdehnung in mindestens einer Richtung gleich oder kleiner 300 nm ist. Vorzugsweise sind die Partikel im Sinne dieser Beschreibung in ihrem mittleren Durchmesser oder in einer ihrer Ausdehnungen in mindestens einer Richtung gleich oder kleiner 100 nm.

Ein Katalysatorträger ist ein Substrat, mit dessen Oberfläche Partikel verbunden werden können, die katalytisch aktiv sind. Die Partikel können direkt mit der Oberfläche verbunden, mit dieser indirekt über Hilfsmaterialien verbunden und / oder in diese eingesenkt sein. Der Katalysatorträger kann durch ein Trägermaterial gebildet sein, das eine Porosität aufweist. Er kann in einem weiteren Ausführungsbeispiel auch aus einer Anzahl von Einzelkörpern gegeben sein, zwischen denen sich Hohlräume befinden und somit eine Porosität gegeben ist. Beispielsweise können die Einzelkörper einzelne Körner, Kugeln oder anders geformte Körper sein, die in einem vorbestimmten Muster zueinander angeordnet sind, beispielsweise in einer vorbestimmten Gitteranordnung. Die Einzelkörper können auch als Schüttung vorliegen, sodass der Katalysatorträger durch die Schüttung realisiert ist.

Es ist auch möglich, dass der Katalysatorträger durch ein Material gebildet ist, das an wenigstens einer seiner Oberflächen Poren aufweist. Beispielsweise kann ein Katalysatorträger ein Glaskörper, beispielsweise in Form einer Platte sein, der an einer Oberfläche Poren aufweist, die den Katalysatorträger nicht vollständig durchdringen.

Unter hohen Feldüberhöhungen wird ein Bereich von dem zwei- bis einhundertfachen der Anregungsfeldstärke verstanden.

Eine Form der Partikel ist beispielsweise kubisch, stäbchenförmig oder kugelförmig. Stäbchenförmige Partikel können beispielsweise einen polygonalen Querschnitt, beispielsweise einen penta-, hexa-, oder octagonalen Querschnitt aufweisen. Das Verhältnis der Oberfläche eines Partikel zu dessen Volumen ist größer 2.

Das Trägermaterial ist offenporig, wenn wenigstens ein Anteil der Poren an einer Oberfläche des Trägermaterials mündet.

Das Trägermaterial gilt als transparent für die Anregungswellenlänge, wenn Licht mit der Anregungswellenlänge, das durch den erfindungsgemäßen Katalysatorträger hindurchgetreten ist, noch mindestens 50% der Intensität des auf den erfindungsgemäßen Katalysatorträger auftreffenden Lichts, bezogen auf die Anregungswellenlänge, aufweist. Licht ist eine elektromagnetische Strahlung. Ein Trägermaterial gilt auch dann als transparent, wenn das Licht der Anregungswellenlänge eine Eindringtiefe von wenigstens 0,1 mm in das Trägermaterial besitzt, wobei eine Eindringtiefe eine Dicke des Trägermaterials ist, in der mindestens noch 50% der Intensität des auf den erfindungsgemäßen Katalysatorträger auftreffenden Lichts, bezogen auf die Anregungswellenlänge, vorhanden ist. Die Eindringtiefe wird an Trägermaterial bestimmt, das keine Poren aufweist.

Die Anregungswellenlänge ist aus einem Wellenlängenbereich von 100 bis 600 nm, vorzugsweise von 190 bis 600 nm, am bevorzugtesten von 190 bis 400 nm ausgewählt und ist größer als die Partikel.

In weiteren Ausführungen des erfindungsgemäßen belegten Katalysatorträgers (nachfolgend auch kürzer als erfindungsgemäßer Katalysatorträger oder als Katalysatorträger bezeichnet) können auch zwei oder mehr Anregungswellenlängen wählbar sein. Dies ist beispielsweise bei Partikeln unterschiedlicher Form und / oder unterschiedlichen Materials günstig, um eine effektive Anregung zu erreichen.

Der Grad der Belegung der Oberfläche der Poren mit Partikeln kann als Belegungsdichte ausgedrückt werden. Die Belegungsdichte wird als Massenanteil des Materials der Partikel an der Masse des erfindungsgemäßen Katalysatorträgers ausgedrückt und beträgt weniger als 20 Masse-%, vorzugsweise aber weniger als 10 Masse-%, und ist beispielsweise kleiner oder gleich 9, 8, 7 oder 6 Masse-%.

Der erfindungsgemäße Katalysatorträger kann auf einem Trägerelement angeordnet sein. Das Trägerelement kann dabei für Licht der Anregungswellenlänge transparent sein, wodurch die Möglichkeit einer Bestrahlung des erfindungsgemäßen Katalysatorträgers durch das Trägerelement hindurch eröffnet ist. Dies ist beispielsweise dann vorteilhaft, wenn der Katalysatorträger in einem Reaktionsraum anzuordnen ist und eine Strahlungsquelle zur Bereitstellung des Lichts außerhalb des Reaktionsraums anzuordnen ist. Dadurch kann die Strahlungsquelle beispielsweise vor nachteiligen thermischen oder chemischen Einflüssen geschützt werden, die beispielsweise in dem Reaktionsraum vorhanden sind.

In einer günstigen Ausführung ist das Trägermaterial ein Glas.

Ein erfindungsgemäßer belegter Katalysatorträger weist vorzugsweise eine Dicke auf, bei der das Licht der Anregungswellenlänge bis in die maximale Ausdehnung eines katalytisch aktiven Volumens propagieren kann. Ein katalytisch aktives Volumen ist derjenige Anteil des Volumens des Katalysatorträgers, der Poren aufweist, mit Partikeln versehen ist und der für Licht der Anregungswellenlänge sowie für Moleküle eines Gases, eines Gasgemisches oder eines Aerosols oder eines sonstigen Mediums aus einer Umgebung des Katalysatorträgers erreichbar ist. Eine Dicke des Katalysatorträgers beträgt wenigstens 0,1 mm, sie kann jedoch in weiteren Ausführungen des erfindungsgemäßen Katalysatorträgers auch dicker, beispielsweise wenigstens 0,2; 0,25; 0,4 oder 0,5 mm sein.

Die Poren des Trägermaterials können eine einheitliche Porengröße oder verschiedene Porengrößen aufweisen. Die mittlere Porengröße liegt vorzugsweise in einem Bereich von 100 nm bis 1 µm.

Sehr günstig ist es, wenn eine Porosität des Trägermaterials eine Porenhierarchie mindestens mit einer ersten Porengröße und einer zweiten Porengröße aufweist, wobei Poren der zweiten Porengröße an Oberflächen von Poren der ersten Porengröße münden. Durch eine Porenhierarchie sind vorteilhaft längere Verweildauern von Molekülen eines Gases, Gasgemisches und / oder eines Aerosols in dem katalytisch aktiven Volumen erreicht.

Die Poren können auch als sich verengende Hohlräume ausgebildet sein. Beispielsweise können die Poren sich in das Trägermaterial hinein verjüngend, etwa kegelförmig zulaufend ausgebildet sein. Die so ausgebildeten Poren münden ebenfalls an einer Oberfläche des Trägermaterials.

Die erste Porengröße ist durch einen mittleren freien Porendurchmesser von mindestens 500 nm und höchstens 20 µm, vorzugsweise aber von höchstens 10 µm gegeben.

Die zweite Porengröße ist durch einen mittleren freien Porendurchmesser von weniger als 500 nm, vorzugsweise weniger als 300 nm, besser noch weniger als 201 nm und am besten in einem Bereich von 100 bis 200 nm gegeben.

Für die katalytische Umsetzung eines Mediums in der katalytisch aktiven Zone ist eine ausreichende Verweildauer in den Poren erforderlich. Diese ist von der Porengröße und der Strömungsgeschwindigkeit des Mediums abhängig, die wiederum proportional zum Porendurchmesser ist. Über die Poren der ersten Porengröße wird daher eine schnellere Aufnahme in den Katalysatorträger und über die Poren der zweiten Porengröße eine längere Verweildauer in diesem erreicht.

Das Material der Partikel ist vorzugsweise aus einer Gruppe beinhaltend Edelmetalle aus der ersten Nebengruppe des Periodensystems und der Gruppe der Platinmetalle und deren Legierungen sowie Mischungen der Edelmetalle und ihrer Legierungen ausgewählt. Edelmetalle bzw. Platinmetalle sind beispielsweise Gold, Platin und Palladium.

Die Porosität des Trägermaterials in dem katalytisch aktiven Volumen beträgt mindestens 30 Volumenprozent. In weiteren Ausführungen kann die Porosität auch mindestens 40, 50, 60, 70 oder 80 Volumen-% betragen.

Die Aufgabe ist ferner durch ein Verfahren zur Herstellung eines mit Partikeln belegten Katalysatorträgers gelöst. Das Verfahren wird mit den nachstehenden Schritten durchgeführt:
- Bereitstellen eines Katalysatorträgers mit offenen Poren aus einem Trägermaterial, das für wenigstens eine Anregungswellenlänge des Lichts transparent ist, wobei durch die Anregungswellenlänge eine Anregung von Plasmonenresonanz auf Oberflächen der Partikel möglich ist und
- Aufbringen von Partikeln mindestens auf Oberflächenbereiche der Poren.

Der bereitgestellte Katalysatorträger ist vorzugsweise ein erfindungsgemäßer Katalysatorträger.

Zum Aufbringen der Partikel können beispielsweise Verfahren wie Ausfällen, Imprägnieren, die Infiltration des Trägermaterials mit Partikeldispersionen oder eine Infiltration von gelösten Metallprecusoren und anschließende in-situ-Herstellung der Partikel in den Poren verwendet werden. Eine in-situ-Herstellung der Partikel erfolgt mittels Zugabe eines Reduktionsmittels oder durch Infiltration von Nanopartikelkeimen, vorzugsweise aufweisend eine mittlere Größe <10 nm, und einem anschließenden Wachstum der Partikel aus einer Metallprecursorlösung aufgrund heterogener Keimbildung.

Das erfindungsgemäße Verfahren kann außerdem Schritte zur Oberflächenmodifizierung des Trägermaterials für die Anbindung von Partikeln und / oder für das Entfernen unerwünschter Nebenprodukte bei der in-situ-Herstellung der Partikel beinhalten.

Nach einem Aufbringen der Partikel auf die Glasoberfläche oder eine in-situ-Herstellung der Partikel aus den Nanopartikelkeimen kann eine Trocknung des Katalysatorträgers erfolgen.

Ein durch ein erfindungsgemäßes Verfahren erhältlicher Katalysatorträger oder ein anders hergestellter erfindungsgemäßer Katalysatorträger kann vorteilhaft zur Durchführung einer Heterokatalyse verwendet werden. Günstig ist dabei die Möglichkeit, eine katalytische Aktivierung der Partikel durch eine Bestrahlung mittels Licht der Anregungswellenlänge und der Anregung von Plasmonenresonanzen an der Oberfläche der Partikel zu bewirken. Die Aktivierung kann dadurch effizient und lokal erfolgen.

Insbesondere kann ein so erhältlicher Katalysatorträger oder ein anders hergestellter erfindungsgemäßer Katalysatorträger zur katalytischen Umsetzung von Schadstoffen in einem Medium verwendet werden. Ein Medium ist ein Gas, ein Gasgemisch, ein Aerosol oder deren Gemische, Umsetzungen in organischen Lösungen oder Ölen.

Die Aufgabe wird auch in einem Verfahren zur Durchführung mindestens einer katalytischen Reaktion von Bestandteilen eines Mediums gelöst. Das Verfahren wird mit den folgenden Schritten durchgeführt:
- Bereitstellen eines mit Partikeln belegten Katalysatorträgers erhältlich durch ein erfindungsgemäßes Verfahren oder eines anders hergestellten erfindungsgemäßen Katalysatorträgers
- Bereitstellen des Mediums,
- Inkontaktbringen des Mediums mit dem Katalysatorträger und Beleuchten des Katalysatorträgers mit der Anregungswellenlänge.

Bestandteile des Mediums, die einer katalytischen Reaktion zugeführt werden sollen, sind chemische Verbindungen, beispielsweise organische Verbindungen wie Fettsäuren, Fette (Triacylglycerine) mit und ohne Mehrfachbindungen, (Poly-)Acrylamide, sowie anorganische Verbindungen wie Kohlenmonoxid und Kohlensäure.

Das Inkontaktbringen des Mediums mit dem Katalysatorträger und Beleuchten des Katalysatorträgers mit der Anregungswellenlänge kann gleichzeitig oder nacheinander erfolgen. Es kann auch erst beleuchtet und dann in Kontakt gebracht werden.

Die Anregungswellenlänge ist aus einem Wellenlängenbereich von 100 bis 600 nm, vorzugsweise von 190 bis 600 nm, am bevorzugtesten von 190 bis 400 nm ausgewählt.

In weiteren Ausführungen des erfindungsgemäßen belegten Katalysatorträgers können auch zwei oder mehr Anregungswellenlängen ausgewählt sein. Dies ist beispielsweise bei Partikeln unterschiedlicher Form und / oder unterschiedlichen Materials günstig, um eine effektive Anregung zu erreichen.

Eine Bestrahlung des erfindungsgemäßen Katalysatorträgers kann von einer Seite des Katalysatorträgers oder von mehreren Seiten her erfolgen. So kann der Katalysatorträger beispielsweise eine Platte sein, die auf einer ihrer Seitenflächen Poren aufweist. Eine Bestrahlung kann direkt auf die Seitenfläche mit den Poren erfolgen. Es ist auch möglich, dass eine Bestrahlung von der anderen Seitenfläche her erfolgt. Dabei dringt Licht der Anregungswellenlänge vorzugsweise so tief in den Katalysatorträger ein, dass durch das Licht die Poren mit den Partikeln erreicht werden und auf den Oberflächen der Partikel Plasmonenresonanz angeregt wird. Die Bestrahlung kann auch auf beide Seitenflächen gerichtet sein.

Die Betriebsparameter wie beispielsweise einer Temperatur in einem Reaktionsraum und / oder des Mediums, Druck, Strömungsgeschwindigkeiten und Volumenströme sind vorzugsweise regel- und / oder steuerbar. Dabei können Messwerte beispielsweise von Sensoren verwendet werden, durch die Eigenschaften des Mediums wie ein aktueller Schadstoffgehalt oder eine Schadstoffzusammensetzung erfasst werden. Diese Messwerte können beispielsweise dazu genutzt werden, um eine gewünschte mittlere Verweildauer von umzusetzenden Molekülen an den Partikeln einzustellen.

Es ist auch möglich, die Betriebsparameter anhand von Versuchen oder praktischen Erfahrungen festzulegen.

Es ist möglich, bei der Durchführung der oben genannten erfindungsgemäßen Verfahren neben einer Anregung durch Licht zusätzlich optional oder obligatorisch eine thermische Anregung durchzuführen. Dazu kann eine Temperatur des Mediums oder des Katalysatorträgers entsprechend geregelt werden.

Es ist auch möglich, dass neben einer Anregung durch Licht eine thermische Anregung dadurch erfolgt, dass Lichtintensitäten verwendet werden, durch die eine thermische Anregung der Partikel zusätzlich zur Anregung einer Plasmonenresonanz bewirkt wird.

Auch kann eine Bestrahlung des Katalysatorträgers mit einer zweiten Wellenlänge erfolgen, die vorzugsweise aus einem Wellenlängenbereich von 300 bis 600 nm ausgewählt ist.

Die Verfahrensparameter sind hinsichtlich des herrschenden Drucks, der Temperatur sowie der Strömungsgeschwindigkeit des Mediums auf die Leistungsfähigkeit des erfindungsgemäßen Katalysatorträgers angepasst. Die Leistungsfähigkeit des Katalysatorträgers ergibt sich beispielsweise aus dessen Ausdehnung, der Porosität, der Belegungsdichte, dem Material und der Form der Partikel sowie der Anregungswellenlänge und deren Intensität. Zur Einstellung und Überwachung sowie zur Steuerung der erfindungsgemäßen Verfahren können geeignete Sensoren vorhanden sein, die mit einer Steuerungseinheit zur Steuerung der Verfahrensabläufe und Betriebsparameter signaltechnisch verbunden sind.

Eine Anwendung der Erfindung liegt beispielsweise in der Behandlung von Abluft von Küchen, Bratereien und Imbissständen. Die meist fetthaltige Abluft verursacht nicht nur unangenehme Gerüche, sondern hinterlässt in den Abluftkanälen fetthaltige Ablagerungen. Diese entstehen insbesondere dann, wenn die Temperatur der Abluft sinkt und diejenigen chemischen Verbindungen nicht mehr durch die Abluft transportiert werden, für deren Transport höhere Temperaturen erforderlich sind. Diese fetthaltigen Ablagerungen sorgen nicht nur für einen hohen Reinigungsaufwand, sondern stellen auch eine ganz erhebliche potentielle Brandquelle dar. Außerdem können auf den organischen Ablagerungen Bakterien und Pilze wachsen, sodass auch aus hygienischer Sicht eine weitgehende Vermeidung solcher Ablagerungen erwünscht ist. Mittels des erfindungsgemäßen Verfahrens zur Durchführung mindestens einer katalytischen Reaktion von Bestandteilen eines Mediums bzw. des erfindungsgemäßen Verfahrens zur katalytischen Umsetzung von Schadstoffen in einem Medium ist es ermöglicht, abgelagerte bzw. sich ablagernde chemische Verbindungen wie (Poly-) Acrylamide, Fette und Fettsäuren in kleinere, d.h. kurzkettige, chemische Verbindungen umzusetzen, die dann wieder durch die Abluft abtransportiert werden können. Besonders günstig ist, wenn eine Umsetzung so weit erfolgt, dass keine oder nur sehr geringe Mengen solcher Verbindungen in der Abluft verbleiben, die unangenehme Gerüche verursachen und / oder gesundheitsschädlich sind.

Die Erfindung wird nachfolgend anhand von Abbildungen näher erläutert. Es zeigen:
- Fig. 1: ein erstes Ausführungsbeispiel eines erfindungsgemäßen Katalysatorträgers mit einer zugeordneten Strahlungsquelle,
- Fig. 2: ein zweites Ausführungsbeispiel eines erfindungsgemäßen Katalysatorträgers in Schnittdarstellung und
- Fig. 3: ein drittes Ausführungsbeispiel eines erfindungsgemäßen Katalysatorträgers.

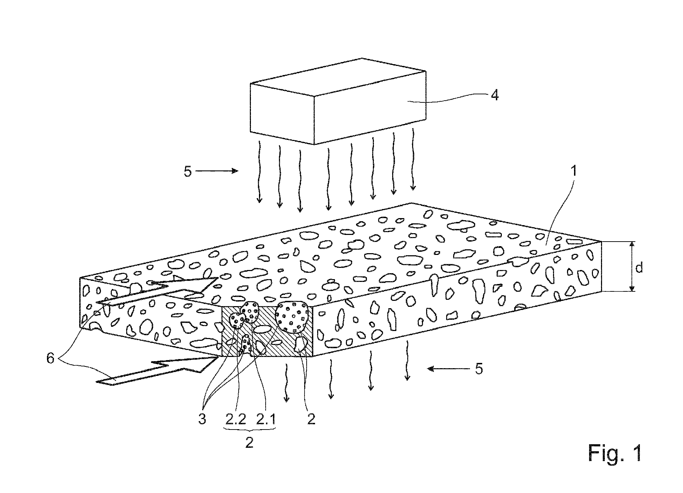

Die wesentlichen Elemente eines erfindungsgemäßen Katalysatorträgers 1 sind in der Fig. 1 mit dem Katalysatorträger 1, Poren 2 und Partikeln 3 schematisch und vereinfachend gezeigt.

Gleiche Bezugszeichen bezeichnen gleiche technische Elemente.

Das Trägermaterial des Katalysatorträgers 1 ist ein poröses Glas mit einer offenen Porosität von 80 Volumen-%. Auf Oberflächen der Poren 2 sind Partikel 3 in Form einzelner Goldpartikel vorhanden. Die Partikel 3 haben eine mittlere Größe von weniger als 100 nm und sind kubisch geformt (angedeutet gezeigt). Die Poren 2 weisen einen mittleren freien Porendurchmesser von 800 nm auf. In dem Teilausschnitt ist eine Pore einer ersten Porengröße 2.1 gezeigt, die mit einer Pore einer zweiten Porengröße 2.2 in Verbindung steht. Durch diese beiden Poren 2.1, 2.2 ist eine Porenhierarchie gebildet. Die Pore einer ersten Porengröße 2.1 weist einen freien Porendurchmesser von 600 nm auf und mündet an der Oberfläche des Katalysatorträgers 1. In die Pore einer ersten Porengröße 2.1 mündet eine Pore einer zweiten Porengröße 2.2. Deren freier Durchmesser beträgt 150 nm. Auch auf den Oberflächen der Pore der ersten Porengröße 2.1 und der Pore der zweiten Porengröße 2.2 sind Partikel 3 vorhanden. Der Katalysatorträger 1 weist eine Dicke d von 0,2 mm auf.

Über dem Katalysatorträger 1 ist eine Strahlungsquelle 4 zur Bereitstellung und gerichteten Abstrahlung eines Lichts 5 einer Anregungswellenlänge angeordnet. Der Katalysatorträger 1 ist von einem gasförmigen Medium 6 (durch Pfeil symbolisiert) umspült, das Schadstoffe in Form eines Gemisches von organischen Verbindungen wie Fetten und Fettsäuren enthält. Das Trägermaterial ist für die Anregungsstrahlung transparent. In Fig. 1 ist schematisch dargestellt, dass eine Intensität des durch den Katalysatorträger 1 hindurchgetretenen Anteils des Lichts 5 mit der Anregungswellenlänge (ein geschweifter Pfeil) 50% der Intensität des von der Strahlungsquelle 4 kommenden und auf den Katalysatorträger 1 einfallenden Lichts 5 (zwei geschweifte Pfeile) beträgt. Der Katalysatorträger 1 ist für das Licht 5 mit der Anregungswellenlänge transparent. Ein Teil des einfallenden Lichts 5 trifft auf die Partikel 3 auf und regt an Oberflächen einiger Partikel 3 Plasmonenresonanz an.

Durch das Volumen des porösen Katalysatorträgers 1 ist ein katalytisch aktives Volumen gebildet.

Gelangen Moleküle der organischen Verbindungen mit den Partikeln 3 in Kontakt, kann durch die Plasmonenresonanzen eine katalytische Reaktion und katalytische Umsetzung der organischen Verbindungen erfolgen.

Die Verfahrensparameter sind hinsichtlich des herrschenden Drucks, der Temperatur sowie der Strömungsgeschwindigkeit des Mediums 6 an die Leistungsfähigkeit des erfindungsgemäßen Katalysatorträgers 1 angepasst.

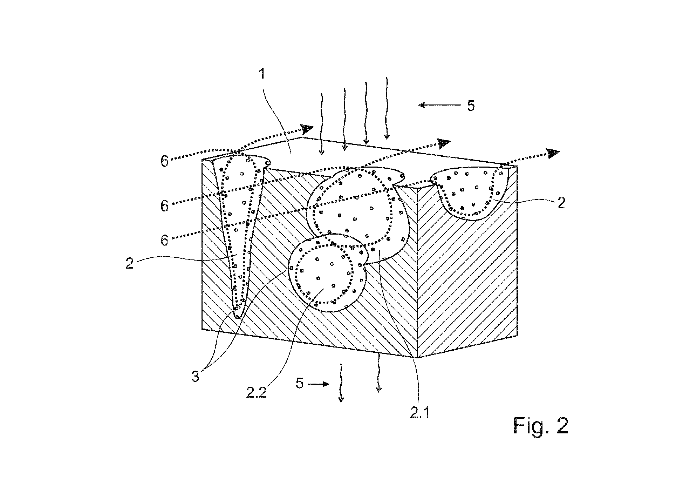

In der Fig. 2 sind schematisch drei Grundtypen von Poren 2 gezeigt. Alle Poren 2 weisen an ihren Oberflächen Partikel 3 auf. An der rechts gezeigten Außenkante des geschnitten gezeigten Katalysatorträgers 1 ist eine Pore 2 angeschnitten, die einen runden Hohlraum bildet, mit keiner anderen Pore 2 in Verbindung steht und an einer Oberfläche des Katalysatorträgers 1 mündet. Ein Strömungsverlauf von Anteilen des Mediums 6 durch die Pore 2 ist durch Pfeile sehr vereinfacht gezeigt. Der Strömungsverlauf lässt erkennen, dass die Pore 2 recht schnell von dem Medium 6 durchströmbar ist und somit eine Verweildauer von Molekülen der organischen Verbindungen an den Partikeln 3 relativ kurz ist. Der Katalysatorträger 1 ist durch ein Glas gebildet, an dessen einer Oberfläche die Poren 2 münden. Die anderen Oberflächen des Katalysatorträgers 1 sind frei von Poren 2.

In der Mitte ist eine Pore einer ersten Porengröße 2.1 gezeigt, die mit einer Pore einer zweiten Porengröße 2.2 in Verbindung steht. Die Pore einer zweiten Porengröße 2.2 ist dabei tiefer in dem Trägermaterial eingebettet als die Pore einer ersten Porengröße 2.1. Durch die beiden Poren 2.1, 2.2 ist eine Porenhierarchie gebildet. Der gezeigte Strömungsverlauf lässt erkennen, dass das Medium 6 länger zum Durchströmen der beiden Poren 2.1, 2.2 benötigt als durch die oben zu Fig. 2 beschrieben Pore 2. Das ist nicht nur durch einen möglicherweise längeren zu durchströmenden Weg, sondern insbesondere auf Strömungswiderstände zurückzuführen, die durch die Poren 2.1, 2.2 verursacht sind. Je kleiner die Poren sind, desto größer ist der Strömungswiderstand. Durch die Poren der ersten Porengröße 2.1 wird das Medium 6 verhältnismäßig schnell vom Katalysatorträger aufgenommen, während es in den Poren der zweiten Porengröße 2.2 verhältnismäßig lange im Katalysatorträger verweilt. Es wird so vorteilhaft eine schnelle Aufnahme und eine lange Verweildauer des Mediums erreicht.

Auf der linken Seite des Katalysatorträgers 1 ist eine Pore 2 dargestellt, die kegelförmig verjüngend in das Trägermaterial ragt. Durch die zunehmende Verengung ist insbesondere im Bereich der Spitze der Pore 2, in dem der Strömungsverlauf umgelenkt ist, eine längere Verweildauer der Moleküle an den Partikeln 3 erreicht.

Das Licht 5 der Anregungswellenlänge durchdringt den Katalysatorträger 1, wobei mindestens 50% der ursprünglichen Intensität erhalten bleibt. Dieser Umstand ist wieder durch die jeweilige Anzahl der geschweiften Pfeile symbolisiert.

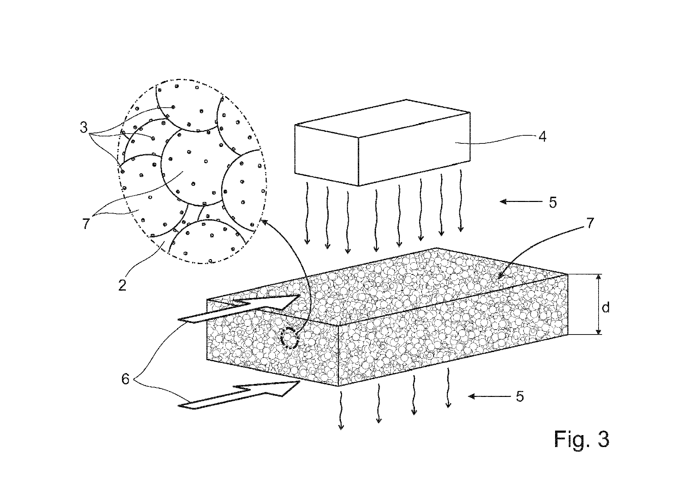

In der Fig. 3 ist ein Katalysatorträger 1 gezeigt, dessen Trägermaterial Glasperlen 2 sind und der aus einer Schüttung der Glasperlen 2 gebildet ist. Zwischen den Glasperlen 7 sind Poren 2 durch die Abstände der Glasperlen 7 zueinander gebildet. In dem vergrößert dargestellten Ausschnitt sind die Glasperlen 7 und die Partikel 3 gut zu erkennen. Zwischen den Glasperlen 7 sind die Poren 2 vorhanden. Hinsichtlich aller anderen Elemente entspricht das dritte Ausführungsbeispiel dem in Fig. 1 gezeigten ersten Ausführungsbeispiel.

### Bezugszeichen

- 1: Katalysatorträger
- 2: Poren
- 2.1: Poren erster Porengröße
- 2.2: Poren zweiter Porengröße
- 3: Partikel
- 4: Strahlungsquelle
- 5: Licht
- 6: Medium
- 7: Glasperle
- d: Dicke

## Patentansprüche

1. Mit katalytisch wirksamen Partikeln (3) belegter Katalysatorträger (1), wobei das Material und die Form der Partikel (3) dazu geeignet ist, bei Einwirkung von Licht (5) einer bestimmten Anregungswellenlänge und Intensität auf der Oberfläche der Partikel (3) Plasmonenresonanz und hohe Feldüberhöhungen hervorzurufen,
**dadurch gekennzeichnet, dass**
- der Katalysatorträger (1) aus einem Trägermaterial besteht, das für die Anregungswellenlänge transparent ist und
- durch das Trägermaterial eine Porosität bereitgestellt ist und die Porosität offenporig ist, wobei freie Oberflächen der Poren (2) mindestens bereichsweise mit Partikeln (3) belegt sind.

2. Katalysatorträger (1) nach Anspruch 1, **dadurch gekennzeichnet,**
**dass** das Trägermaterial ein Glas ist.

3. Katalysatorträger (1) nach Anspruch 1 oder 2, **dadurch gekennzeichnet,**
**dass** der Katalysatorträger (1) eine solche Dicke (d) aufweist, dass das Licht (5) bis in die maximale Ausdehnung eines katalytisch aktiven Volumens des Katalysatorträgers (1) propagieren kann, wobei das katalytisch aktive Volumen derjenige Anteil des Volumens des Katalysatorträgers (1) ist, der Poren (2) aufweist, mit Partikeln (3) versehen ist und der für das Licht (5) der Anregungswellenlänge sowie für Moleküle eines Mediums (6) aus einer Umgebung des Katalysatorträgers (1) erreichbar ist.

4. Katalysatorträger (1) nach einem der Ansprüche 1 bis 3, **dadurch gekennzeichnet, dass** eine Porosität des Trägermaterials eine Porenhierarchie mindestens mit Poren einer ersten Porengröße (2.1) und mit Poren einer zweiten Porengröße (2.2) aufweist, wobei Poren der zweiten Porengröße (2.2) an Oberflächen von Poren der ersten Porengröße (2.1) münden.

5. Katalysatorträger (1) nach Anspruch 4, **dadurch gekennzeichnet,**
**dass** die Poren einer ersten Porengröße (2.1) mittlere freie Porendurchmesser von mindestens 500 nm und höchstens 10 µm aufweisen.

6. Katalysatorträger (1) nach Anspruch 4 oder 5, **dadurch gekennzeichnet,**
**dass** die Poren einer zweiten Porengröße (2.2) mittlere freie Porendurchmesser von weniger als 500 nm aufweisen.

7. Katalysatorträger (1) nach einem der Ansprüche 1 bis 6, **dadurch gekennzeichnet,**
**dass** das Material der Partikel (3) aus einer Gruppe beinhaltend Edelmetalle aus der ersten Nebengruppe des Periodensystems und der Gruppe der Platinmetalle und deren Legierungen sowie Mischungen der Edelmetalle und ihrer Legierungen ausgewählt ist.

8. Katalysatorträger (1) nach einem der Ansprüche 1 bis 7, **dadurch gekennzeichnet,**
**dass** die Porosität des Trägermaterials in dem katalytisch aktiven Volumen mindestens 30 Volumenprozent beträgt.

9. Katalysatorträger (1) nach einem der Ansprüche 1 bis 8, **dadurch gekennzeichnet,**
**dass** die Anregungswellenlänge aus einem Wellenlängenbereich von 100 bis 600 nm ausgewählt ist.

10. Verfahren zur Herstellung eines mit Partikeln (3) belegten Katalysatorträgers (1) mit den Schritten
- Bereitstellen eines Katalysatorträgers (1) mit offenen Poren (2) aus einem Trägermaterial, das für wenigstens eine Anregungswellenlänge eines Lichts (5) transparent ist, wobei durch die Anregungswellenlänge eine Anregung von Plasmonenresonanz auf Oberflächen der Partikel (3) möglich ist und
- Aufbringen von Partikeln (3) mindestens auf Oberflächenbereiche der Poren (2).

11. Verwendung eines Katalysatorträgers (1) erhältlich durch ein Verfahren nach Anspruch 10 oder eines Katalysatorträgers (1) nach einem der Ansprüche 1 bis 9 zur Durchführung einer Heterokatalyse.

12. Verwendung eines Katalysatorträgers (1) nach einem der Ansprüche 1 bis 9 zur katalytischen Umsetzung von Schadstoffen in einem Medium (6).

13. Verfahren zur Durchführung mindestens einer katalytischen Reaktion von Bestandteilen eines Mediums (6) mit den Schritten:
- Bereitstellen eines mit Partikeln (3) belegten Katalysatorträgers (1) erhältlich durch ein Verfahren nach Anspruch 10 oder eines Katalysatorträgers (1) nach einem der Ansprüche 1 bis 9,
- Bereitstellen des Mediums (6),
- Inkontaktbringen des Mediums (6) mit dem Katalysatorträger (1) und Beleuchten des Katalysatorträgers (1) mit Licht (5) der Anregungswellenlänge.

14. Verfahren nach einem der Ansprüche 10 bis 13, **dadurch gekennzeichnet, dass** neben einer Anregung durch Licht (5) zusätzlich eine thermische Anregung der Partikel (3) durchgeführt wird.

15. Verfahren nach Anspruch 14, **dadurch gekennzeichnet,**
**dass** die thermische Anregung der Partikel (3) durch eine Bestrahlung des Katalysatorträgers (1) mit einer zweiten Wellenlänge erfolgt und die zweite Wellenlänge aus einem Wellenlängenbereich von 300 bis 600 nm ausgewählt ist.
